(19) Europäisches Patentamt
European Patent Office
Office européen des brevets

(11) EP 0 568 898 B1

# (12) EUROPÄISCHE PATENTSCHRIFT

(45) Veröffentlichungstag und Bekanntmachung des Hinweises auf die Patenterteilung:
**01.04.1998 Patentblatt 1998/14**

(51) Int. Cl.$^6$: **C07D 335/06**, C07D 337/08, C07D 333/54, A61K 31/38

(21) Anmeldenummer: **93106773.0**

(22) Anmeldetag: **27.04.1993**

(54) **Benzothiepin-, Benzothiopyran- und Benzothiophenderivate, deren Herstellung und Verwendung als Heilmittel**

Benzothiepine-, benzothiopyrane- and benzothiophenyle derivatives, their preparation and use as medicine

Dérivés des benzothiépine, benzothiopyranne et benzothiophényle, leur préparation et utilisation comme médicament

(84) Benannte Vertragsstaaten:
**AT BE CH DE DK ES FR GB GR IE IT LI LU MC NL PT SE**

(30) Priorität: **07.05.1992 CH 1465/92**

(43) Veröffentlichungstag der Anmeldung:
**10.11.1993 Patentblatt 1993/45**

(73) Patentinhaber:
**F. HOFFMANN-LA ROCHE AG**
**4002 Basel (CH)**

(72) Erfinder:
- **Klaus, Michael**
  **W-7858 Weil/Rhein (DE)**
- **Mohr, Peter**
  **CH-4054 Basel (CH)**

(74) Vertreter:
**Grossner, Lutz, Dr. et al**
**F.Hoffmann-La Roche AG**
**Patent Department (PLP),**
**124 Grenzacherstrasse**
**4070 Basel (CH)**

(56) Entgegenhaltungen:
**EP-A- 0 290 130**
**EP-A- 0 350 846**
**WO-A-85/00806**
**GB-A- 2 119 801**
**US-A- 4 678 793**
**US-A- 4 833 254**
**US-A- 4 980 369**

- **FUNDAMENTAL & APPLIED TOXICOLOGY, Band 14, Nr. 2, 1990; M.R. FRIERSON et al., Seiten 408-428**
- **CANCER RESEARCH, Band 51, Nr. 18, 15 September 1991; J.M. LEHMANN et al., Seiten 4804-4809**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 568 898 B1

Printed by Xerox (UK) Business Services
2.15.12/3.4

## Beschreibung

Die vorliegende Erfindung betrifft neue heterocyclische Verbindungen der Formel

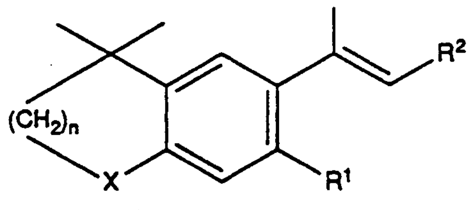

I

in der X -S-,-SO-oder $-SO_2-$;

$R^1$ $C_{7-10}$-Alkyl oder $C_{7-10}$-Alkoxy;
$R^2$ einen Rest der Formeln

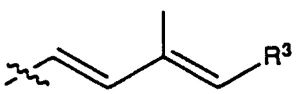

(a)

oder

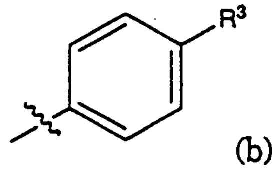

(b)

$R^3$ Carboxy oder $C_{1-6}$-Alkoxycarbonyl; und n 1,2 oder 3 darstellt; und Salze von Carbonsäuren der Formel I.

Die Erfindung betrifft weiterbin pharmazeutische Präparate auf der Basis von Verbindungen der Formel I oder deren Salzen sowie ein Verfahren zur Herstellung der Verbindungen I.

Der hier verwendete Ausdruck "$C_{1-6}$" bezeichnet Gruppen mit bis zu 6 Koblenstoffatomen; Bevorzugt sind Gruppen mit 1-4 Koblenstoffatomen, wie beispielsweise Methyl, Aethyl, Isopropyl oder 2-Methylpropyl.

Der Ausdruck $C_{7-10}$-Alkyl und $C_{7-10}$-Alkoxy bezeichnet Alkyl- und Alkoxygruppen mit 7-10 Kohlenstoffatomen, wie Heptyl, Octyl, Nonyl und Decyl.

Eine bevorzugte Gruppe von Verbindungen der Formel I sind diejenigen, in denen $R^1$ Heptyl, Octyl, Heptyloxy oder Octyloxy; $R^3$ Carboxyl und X $=SO_2$ ist.

Die Verbindungen der Formel I können dadurch erhalten werden, dass man

a) eine Verbindung der Formel

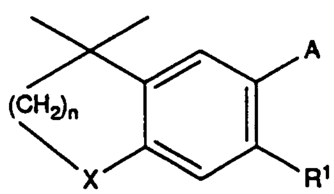

II

mit einer Verbindung der Formeln

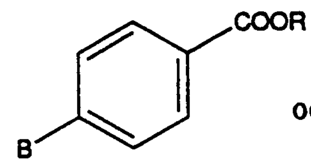

oder

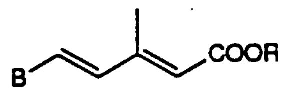

IV

III

umsetzt, wobei R $C_{1-6}$-Alkyl ist und entweder A eine Triphenylphosphoniumäthylgruppe

$CH_3CH-P[Q]_3^+Y^-$

oder Dialkoxyphosphinyläthylgruppe

$CH_3CH-PO(OR)_2$

und B Formyl ist; oder A Acetyl und B eine Triphenylmethylphosphoniumgruppe $-CH_2-P[Q]_3^+Y^-$ oder eine Dialkoxyphosphinylmethylgruppe $-CH_2-PO(OR)_2$ ist; wobei Q Phenyl ist; oder dass man

b) eine Verbindung der Formel

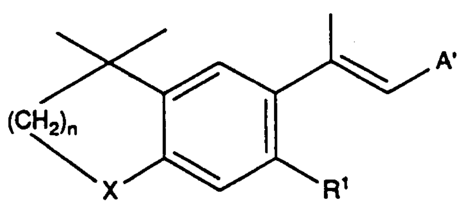

V

mit einer Verbindung der Formel

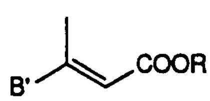

VI

wobei entweder A' eine Triphenylmethylphosphoniumgruppe $-CH_2-\overset{+}{P}[Q]_3Y^-$ oder eine Dialkoxyphosphinylmethylgruppe $-CH_2-PO(OR)_2$ und B' Formyl ist; oder A' Formyl und B' eine Triphenylmethylphosphoniumgruppe $-CH_2-\overset{+}{P}[Q]_3Y^-$ oder eine Dialkoxyphosphinylmethylgruppe $-CH_2PO(OR_2)$ ist, und wobei n, Q und R die oben angegebene Bedeutung haben, zu einer Verbindung der Formel I, in der $R^3$ -COOR ist, umsetzt und gewünschtenfalls die Estergruppe -COOR verseift und die erhaltene Carbonsäure als solche oder als Salz isoliert; und/oder eine erhaltene Verbindung der Formel I, in der X -S- ist, zu einer Verbindung der Formel I, in der X -SO- oder $-SO_2-$ ist, oxidiert.

Die Umsetzung der Verbindung II mit den Verbindungen III oder IV und die Umsetzung der Verbindung V mit der Verbindung VI kann nach an sich bekannten Methoden der Wittig- bzw. Horner-Reaktion durchgeführt werden.

Die Umsetzung von Verbindungen mit Triphenylphosphoniumgruppen (Wittig-Reaktion) kann in Gegenwart eines säurebindenden Mittels, z.B. einer starken Base, wie z.B. Butyllithium, Natriumhydrid oder dem Natriumsalz von Dimethylsulfoxyd, vornehmlich aber in Gegenwart eines gegebenenfalls durch $C_{1-6}$ Alkyl substituierten Aethylenoxyds wie 1,2-Butylenoxyd, gegebenenfalls in einem Lösungsmittel, z.B. in einem Aether, wie Diäthyläther oder Tetrahydrofuran,

oder in einem aromatischen Kohlenwasserstoff, wie Benzol, in einem zwischen der Raumtemperatur und dem Siedepunkt des Reaktionsgemisches liegenden Temperaturbereich erfolgen.

Beispiele von Anionen $Y^-$ im Wittig-Reagenz sind $Cl^-$, $Br^-$, $HSO_4^-$ und Tosylat.

Die Umsetzung von Verbindungen mit Dialkoxyphosphinylgruppen (Horner-Reaktion) können in Gegenwart einer Base und, vorzugsweise, in Gegenwart eines inerten organischen Lösungsmittels, z.B. in Gegenwart von Natriumhydrid in Benzol, Toluol, Dimethylformamid, Tetrahydrofuran, Dioxan oder 1,2-Dimethoxyalkan, oder auch Natriumalkoholat in einem Alkanol, z.B. Natriummethylat in Methanol, in einem zwischen 0° und dem Siedepunkt des Reaktionsgemisches liegenden Temperaturbereich durchgeführt werden.

Ein so erhaltener Carbonsäureester der Formel I kann in an sich bekannter Weise, z.B. durch Behandeln mit Alkalien, insbesondere durch Behandeln mit wässriger alkoholischer Natron- oder Kalilauge in einem zwischen Raumtemperatur und dem Siedepunkt des Reaktionsgemisches liegenden Temperaturbereich hydrolysiert werden.

Die so erhaltene Carbonsäure der Formel I kann in an sich bekannter Weise als solche isoliert werden oder als Salz, z.B. als Alkalisalz, insbesondere als Na- oder K-Salz isoliert werden.

Eine Verbindung der Formel I, in der X für -S- steht, kann mit an sich bekannten Methoden zu einer Verbindung der Formel I, in der X für $-SO^-$ oder $-SO_2^-$ steht, oxidiert werden. Die Oxidation kann mit Oxidationsmitteln wie Periodaten, z.B. $NaJO_4$ oder mit organischen Persäuren, wie m-Chlorperbenzoesäure vorgenommen werden. Bei der Oxidation mit organischen Persäuren setzt man etwa ein Aequivalent Persäure ein, um eine Sulfoxidverbindung (X=SO) zu erhalten, wogegen die Verwendung von zwei Aequivalenten Persäure zu Sulfonen ($X=SO_2$) führt.

Die Verbindungen der Formel I können als Doppelbindungsisomere vorliegen. Bei der Herstellung fallen sie mehrheitlich in der trans- bzw. alltrans-Form an. Gegebenenfalls anfallende cis-Isomere können in an sich bekannter Weise, falls erwünscht, abgetrennt werden.

Die Ausgangsstoffe der Formeln II-VI sind bekannt oder können in Analogie zu bekannten oder in den nachstehenden Beispielen beschriebenen Methoden hergestellt werden.

Die Dokumente WO-A-8 500 806, US-A-4 678 793, GB-A-2 119 801, EP-A-O 350 846, US-A-4 833 254, Fundam.Appl.Toxicol.Bd.14, Nr.2, 1990,408-428, Cancer Res, Bd. 51, Nr.18,1991,4804-4809 beschreiben strukturähnliche Verbindungen, in denen der $C_{7-10}$-Alkyl oder Alkoxy-Substituent $R^1$ der vorliegenden Verbindungen fehlt oder Methyl oder, im Fall des Dokuments EP-A-O 350 846, $C_{1-6}$-Alkyl oder Alkoxy bedeutet. Die Dokumente EP-A-0 2909 130 und US-A-4 980 369 beschreiben Verbindungen, die den das Strukturelement (b) enthaltenden vorliegenden Verbindungen strukturähnlich sind und anstelle des Alkenylrestes, der die Ringsysteme miteinander verbindet, einen Ethinylen-Rest aufweisen.

Die Verbindungen der Formel I sind Hemmstoffe für den Retinsäure-a-Rezeptor (RARa-Rezeptor). Es wurde gefunden, dass die Verbindungen der Formel I Retinoid-induzierte fötale Missbildungen unterdrücken. Die Verbindungen der Formel I können daher zur Verhütung von teratogenen Effekten verwendet werden, die bei der therapeutischen Anwendung von Retinoiden auftreten können.

Die Verbindungen der Formel I können weiterhin zur Behandlung und Verhütung von Krankheitszuständen verwendet werden, die durch eine Ueberregulation des RAR$\alpha$-Rezeptors bedingt sein können. In Betracht kommen hier Autoimmunerkrankungen, oder andere Erkrankungen mit einer stark immunologischen Komponente wie z.B. Psoriasis oder andere dermatologische Affektionen.

Die Verbindungen der Formel I und deren Salze können in Form pharmazeutischer Präparate Anwendung finden.

Die zur systemischen Anwendung dienenden Präparate können z.B. dadurch hergestellt werden, dass man eine Verbindung der Formel I oder ein Salz davon als wirksamen Bestandteil nichttoxischen, inerten, an sich in solchen Präparaten üblichen festen oder flüssigen Trägern zufügt.

Die Mittel können enteral, parenteral oder topisch verabreicht werden. Für die enterale Applikation eignen sich z.B. Mittel in Form von Tabletten, Kapseln, Dragées, Sirupen, Suspensionen, Lösungen und Suppositorien. Für die parenterale Applikation sind Mittel in Form von Infusions- oder Injektionslösungen geeignet.

Für die enterale und parenterale Verabreichung können die Verbindungen der Formel I in Mengen von etwa 1-100 mg, vorzugsweise 5-30 mg/Tag, an Erwachsene verabreicht werden.

Zur topischen Anwendung werden die Wirkstoffe zweckmässig in Form von Salben, Tinkturen, Crèmen, Lösungen, Lotionen, Sprays, Suspensionen und dergleichen verwendet. Bevorzugt sind Salben und Crèmen sowie Lösungen. Diese zur topischen Anwendung bestimmten Präparate können dadurch hergestellt werden, dass man die Verfahrensprodukte als wirksamen Bestandteil nichttoxischen, inerten, für topische Behandlung geeigneten, an sich in solchen Präparaten üblichen festen oder flüssigen Trägern zumischt.

Für die topische Anwendung sind zweckmässig ca. 0,1-5%ige, vorzugsweise 0,3-2%ige Lösungen, sowie ca. 0,1-5%ige, vorzugsweise ca. 0,3-2%ige, Salben oder Crèmen geeignet.

Den Präparaten kann gegebenenfalls ein Antioxydationsmittel, z.B. Tocopherol, N-Methyl-$\gamma$-tocopheramin sowie butyliertes Hydroxyanisol oder butyliertes Hydroxytoluol beigemischt werden.

In den nachfolgenden Beispielen, die die Erfindung weiter erläutern, werden folgende Abkürzungen verwendet:

| | |
|---|---|
| DMF | Dimethylformamid |
| DMSO | Dimethylsulfoxid |
| THF | Tetrahydrofuran |
| RT | Raumtemperatur |
| F.p. | Schmelzpunkt |
| i.V. | im Vakuum |
| EtOEt | Diäthyläther |
| tBuOH | tert.-Butanol |
| AcOEt | Aethylacetat |

Beispiel 1

a) 14.9 g NaH, 50% in Paraffinöl, wurden zweimal mit Pentan gewaschen, am Wasserstrahlvakuum getrocknet und in 60 ml DMF suspendiert. Unter Eiskühlung tropfte man eine Lösung von 64.8 g 3-Heptyloxyphenol in 320 ml DMF hinzu. Nach 45-minütigem Rühren bei 0°C tropfte man langsam eine Lösung von 42.3 g Dimethyl-thiocarbamoylchlorid in 100 ml DMF zu diesem Reaktionsgemisch dazu und liess über Nacht bei Raumtemperatur rühren. Danach wurde auf Eiswasser gegossen, mit 6N Salzsäure angesäuert und mit AcOEt extrahiert. Die organische Phase wurde mit Wasser, gesättigter $NaHCO_3$-Lösung und $H_2O$ gewaschen, über Natriumsulfat getrocknet und eingedampft. Nach Filtration des Rohproduktes über eine Kieselgelsäule (Eluierungsmittel Hexan/AcOEt = 4:1) wurden 86.7 g O-(3-Heptyloxyphenyl)dimethylthiocarbamat als schwach gelbes Oel erhalten.

b) 80 g O-(3-Heptyloxyphenyl)dimethylthiocarbamat wurden in 20 g Portionen im Metallbad während 8 Stunden auf 260°C erhitzt. Das so erhaltene S-(3-Heptyloxyphenyl)dimethylthiocarbamat wurde ohne weitere Reinigung in der nächsten Stufe eingesetzt.

c) 10 g $LiAlH_4$ wurden in 200 ml THF suspendiert und bei 0°C tropfenweise mit einer Lösung von 77 g S-(3-Heptyloxyphenyl)dimethylthiocarbamat in 200 ml THF versetzt. Nach 1-stündigem Rühren bei Raumtemperatur tropfte man eine Lösung von 46.5 g 3,3-Dimethylallylbromid in 200 ml THF hinzu und rührte weitere 45 Minuten. Das Reaktionsgemisch wurde anschliessend auf ein Eiswasser/6N Salzsäure-Gemisch gegossen und mit EtOEt extrahiert. Nach Waschen, Trocknen und Eindampfen erhielt man ein dunkles, öliges Rohprodukt, das nach Filtration über eine Kieselgelsäule (Eluierungsmittel Hexan/AcOet = 9:1) 64 g Heptyl m-[(3-methyl-2-butenyl)thio]phenyläther als gelbes Oel ergab.

d) 76.9 g Heptyl m-[(3-methyl-2-butenyl)thio]phenyläther wurden in 1.5 l Toluol gelöst, mit 55 g p-Toluolsulfonsäure versetzt und während 20 Stunden am Wasserabscheider erhitzt. Das abgekühlte Reaktionsgemisch wurde mit AcOEt verdünnt, 2 mal mit verdünnter Natriumbicarbonat-Lösung und Wasser gewaschen, getrocknet und eingedampft. Man erhielt ein gelbes Oel, das zu 3 Teilen aus 7-(Heptyloxy)-3,4-dihydro-4,4-dimethyl-2H-1-benzothiopyran und zu 1 Teil aus 5-(Heptyloxy)-3,4-dihydro-4,4-dimethyl-2H-1-benzothiopyran bestand. Säulenchromatographie (Kieselgel, Eluierungsmittel Hexan/1% AcOEt) ergab 48.6g reines 7-(Heptyloxy)-3,4-dihydro-4,4-dimethyl-2H-1-benzothiopyran als schwach gelbes Oel.

e) 4.8 ml Acetylchlorid wurden in 80 ml Methylenchlorid gelöst und bei 0°C portionenweise mit 9.1g Aluminiumchlorid versetzt. Nach 30-minütigem Rühren bei 0°C tropfte man eine Lösung von 20 g 7-(Heptyloxy)-3,4-dihydro-4,4-dimethyl-2H-1-benzothiopyran in 70 ml Methylenchlorid hinzu, rührte 2 Stunden bei 0°C, goss auf Eiswasser und extrahierte mit Aether. Nach Waschen mit Wasser, Trocknen und Eindampfen kristallisierte man das Rohprodukt aus Hexan um und erhielt 15.5 g 6-Acetyl-7-(heptyloxy)-3,4-dihydro-4,4-dimethyl-2H-1-benzothiopyran in farblosen Kristallen, F.p. 69-71°C.

f) 14 g 6-Acetyl-7-(heptyloxy)-3,4-dihydro-4,4-dimethyl-2H-1-benzothiopyran wurden in 200 ml Chloroform gelöst und bei 0°C tropfenweise mit einer Lösung von 8.5 g m-Chlorperbenzoesäure (85%) in 120 ml Chloroform versetzt. Nach 2-stündigem Rühren bei 0°C wurden weitere 8.5 g m-Chlorperbenzoesäure in 120 ml Chloroform zugetropft. Man rührte das Reaktionsgemisch über Nacht bei 0°C, goss auf Eiswasser/verdünnte Kochsalzlösung und extrahierte mit Methylenchlorid. Die organische Phase wurde 2 mal mit Wasser gewaschen, getrocknet und eingedampft. Nach Filtration des Rohproduktes über eine Kieselgelsäule (Eluierungsmittel Hexan/AcOEt = 4:1) und Umkristallisation aus AcOEt erhielt man 12.5 g 7-(Heptyloxy)-3,4-dihydro-4,4-dimethyl-2H-1-benzothiopyran-6-yl-methylketon-1,1-dioxid in farblosen Kristallen, F.p. 92-93°C.

g) 3 g Natriumhydrid (50% in Mineralöl) wurden zweimal mit Pentan gewaschen, am Wasserstrahlvakuum getrocknet und in 50 ml abs. DMSO suspendiert. Bei Raumtemperatur tropfte man langsam eine Lösung von 19.3 g Diäthyl (4-carbäthoxybenzyl)phosphonat in 100 ml abs. DMSO hinzu. Nach 2-stündigem Rühren bei Raumtemperatur versetzte man tropfenweise mit einer Lösung von 10.5 g 7-(Heptyloxy)-3,4-dihydro-4,4-dimethyl-2H-1-benzothiopyran-6-yl-methylketon-1,1-dioxid in 50 ml abs. DMSO und rührte weitere 2 Stunden bei 40°C. Nach dem Abkühlen goss man das Reaktionsgemisch auf Eiswasser, säuerte mit 2N Salzsäure an und extrahierte mit Essigester. Die organische Phase wurde mit Wasser gewaschen, über Natriumsulfat getrocknet und eingedampft. Der ölige, dunkelorange Rückstand wurde über eine Kieselgelsäule filtriert (Eluierungsmittel Hexan/AcOEt = 2:1) und ergab 13.5g Aethyl p-[(E,Z)-2-[3',4'-Dihydro-4',4'-dimethyl-7'-(heptyloxy)-2'H-1-benzothiopyran-6'-yl]propenyl]benzoat-1'1'-dioxid als gelbes Oel (E/Z-Verhältnis ca. 1:1).

Beispiel 2

13.5 g Aethyl p-[(E,Z)-2-[3',4'-dihydro-4',4'-dimethyl-7'-(heptyloxy)-2'H-1-benzothiopyran-6'-yl]propenyl]benzoat-1',1'-dioxid wurden in 250 ml Aethanol gelöst und mit einer Lösung von 14.6 g Kaliumhydroxid in 100 ml Wasser versetzt. Nach 3-stündigem Rühren bei 50°C goss man das Reaktionsgemisch auf Eiswasser, säuerte mit 3N Salzsäure an und extrahierte mit AcOEt. Die organische Phase wurde mit Wasser gewaschen, getrocknet und eingedampft. Der Rückstand wurde aus Essigester/Hexan umkristallisiert und ergab 10.2g eines E/Z-Gemisches der entsprechenden Säuren. Mittels präparativer HPLC (reverse phase, Eluierungsmittel Hexan/THF = 9:1 + 0.1% Essigsäure) erhielt man nach Umkristallisation aus AcOEt/Hexan 4.1 g p-[(E)-2-[3',4'-Dihydro-4',4'-dimethyl-7'-(heptyloxy)-2'H-1-benzothiopyran-6'-yl]propenyl]benzoesäure-1',1'-dioxid in farblosen Kristallen, F.p. 168-169°C, sowie 5.2 g p-[(Z)-2-[3',4'-Dihydro-4',4'-dimethyl-7'-(heptyloxy)-2'H-1-benzothiopyran-6'-yl]propenyl]benzoesäure-1',1'-dioxid, F.p. 176-178°C.

Die Z-Verbindung kann durch Bestrahlen mit einer Hg-Hochdrucklampe in THF in ein 1:1-Gemisch der E/Z-Isomeren überführt werden, aus dem durch präparative HPLC weitere reine E-Verbindung gewonnen werden kann.

Beispiel 3

a) 13.6 g 6-Acetyl-7-(heptyloxy)-3,4-dihydro-4,4-dimethyl-2H-1-benzothiopyran wurden in 270 ml THF gelöst. Bei -15°C tropfte man 164 ml einer 1 molaren Lösung von Vinylmagnesiumbromid in THF hinzu und rührte bei Raumtemperatur über Nacht. Das Reaktionsgemisch wurde anschliessend auf eiskalte, gesättigte Ammoniumchiorid-Lösung gegossen, mit EtOEt extrahiert, mit Wasser gewaschen, getrocknet und eingedampft. Man erhielt ein gelbbraunes Oel, das sofort in 270 ml Acetonitril gelöst und unter Rühren portionenweise mit 16.3 g Triphenylphosphinhydrobromid versetzt wurde. Nach 2.5-stündigem Rühren des Reaktionsgemisches bei 50°C dampfte man ein, löste den Rückstand in 500 ml Aethanol (80%) und extrahierte mehrfach mit Hexan. Die äthanolische Lösung wurde eingedampft und der Rückstand in Methylenchlorid gelöst. Nach Trocknen über Natriumsulfat dampfte man erneut ein und rührte den schaumigen Rückstand mehrere Stunden mit Hexan, filtrierte den inzwischen kristallinen Niederschlag, wusch mit Hexan und trocknete am Hochvakuum bei 50°C. Man erhielt 26.6 g 3-[(E)-(7-Heptyloxy-3,4-dihydro-4,4-dimethyl-2H-1-benzothiopyran-6-yl)-2-butenyl]triphenylphosphoniumbromid, F.p. 88°C (Zersetzung).

b) 25 g des in a) erhaltenen Phosphoniumsalzes wurden in 250 ml THF gelöst und bei -20°C tropfenweise mit 25 ml Butyllithium, 1.6 molar in Hexan, versetzt. Nach 15 Minuten tropfte man zum rotbraunen Reaktionsgemisch eine Lösung von 6.3g (E)-3-Formyl-crotonsäureäthylester in 30 ml THF und rührte weitere 45 Minuten bei Raumtemperatur. Man goss auf 500 ml eines Methanol/Wasser-Gemisches (6:4), extrahierte mehrfach mit Hexan, wusch die nicht-wässrige Phase 3 mal mit Wasser, trocknete und dampfte ein. Der gelbe, ölige Rückstand wurde in 500 ml Acetonitril gelöst und nach Zugabe von 560 mg Triphenylphosphin mit 28 ml einer 0.125%-igen Lösung von Palladium(II)nitrat in Acetonitril versetzt. Man erwärmte das Gemisch während 5 Stunden auf 50°C, dampfte anschliessend ein und filtrierte das Rohprodukt über eine kurze Kieselgelsäure (Eluierungsmittel Hexan/1% AcOEt). Man erhielt 13.7 g eines gelben Oeles, das aus einem Gemisch der entsprechenden (4Z,6Z)-,(all-E)- und (6Z)-Verbindung bestand. Die weitere Auftrennung erfolgte durch eine Mitteldruckchromatographie mit Lobar-Fertigsäulen (Merck) (Eluierungsmittel Hexan/3% AcOEt) und ergab 6.6 g (all-E)-7-[7-Heptyloxy-3,4-dihydro-4,4-dimethyl-2H-1-benzothiopyran-6-yl]-3-methyl-octa-2,4,6-triensäureäthylester als gelbes Oel.

Beispiel 4

500 mg des in Beispiel 3 erhaltenen (all-E)-Aethylesters wurden in 20 ml Aethanol gelöst und mit einer Lösung von 560 mg Kaliumhydroxid in 10 ml Wasser versetzt. Nach 3 Stunden bei 50°C goss man die klare, gelbe Lösung auf Eiswasser, säuert mit kalter 3N Salzsäure an und extrahierte mit AcOEt. Nach dem Trocknen und Eindampfen kristallisierte man das Rohprodukt aus AcOEt/Hexan um und erhielt 150 mg (all-E)-7-[7-Heptyloxy-3,4-dihydro-4,4-dimethyl-2H-1-benzothiopyran-6-yl]-3-methyl-2,4,6-octatriensäure als gelbe Kristalle, F.p. 158-160°C.

Beispiel 5

Oxidation des in Beispiel 3 erhaltenen (all-E)-Aethylester mit 1 Aequivalent m-Chlorperbenzoesäure bei 0°C in Chloroform als Lösungsmittel ergab (all-E)-7-[7-Heptyloxy-1-oxo-3,4-dihydro-4,4-dimethyl-2H-1-benzothiopyran-6-yl]-3-methyl-octa-2,4,6-triensäureäthylester als gelbes Oel, das durch Hydrolyse in Analogie zu Beispiel 4 in (all-E)-7-[7-Heptyloxy-1-oxo-3,4-dihydro-4,4-dimethyl-2H-1-benzothiopyran-6-yl]-3-methyl-octa-2,4,6-triensäure überführt wurde. Schmelzpunkt 195-197°C (aus AcOEt/Hexan).

Beispiel 6

Oxidation des nach Beispiel 3 erhaltenen (all-E)-Aethylesters mit 2.2 Aequivalenten m-Chlorperbenzoesäure bei 0°C in Chloroform ergab nach Umkristallisation aus Hexan Aethyl (all-E)-7-[7'-(heptyloxy)3',4'-dihydro-4',4'-dimethyl-2'H-1-benzothiopyran-6'-yl]-3-methyl-2,4,6-octatrienoat-1',1'-dioxid, F.p. 105-107°C. Hydrolyse dieser Verbindung in Analogie zu Beispiel 4 ergab nach Umkristallisation aus AcOEt/Hexan (all-E)-7-[7'-(Heptyloxy)-3',4'-dihydro-4',4'-dimethyl-2'H-1-benzothiopyran-6'-yl)-3-methyl-2,4,6-octatriensäure-1',1'-dioxid, F.p. 140-141°C.

Beispiel 7

a) Zu einer Suspension von 4 g Natriumhydrid (50% in Mineralöl) in 50 ml DMF tropfte man bei 0°C eine Lösung von 10 g m-Hydroxybenzaldehyd in 100 ml DMF. Man rührte eine weitere Stunde bei 0°C und tropfte anschliessend eine Lösung von 11.1 g Dimethylthiocarbamoylchlorid in 150 ml DMF hinzu. Man rührte über Nacht bei Raumtemperatur, goss auf Eiswasser, säuerte mit 6N Salzsäure an und extrahierte mit AcOEt. Nach dem Trocknen der organischen Phase, Eindampfen und Filtration des Rohproduktes über eine Kieselgelsäule (Eluierungsmittel Hexan/AcOEt = 3:1) erhielt man 10.2 g O-(3-Formylphenyl)dimethylthiocarbamat als gelbliches Oel. Dieses Produkt wurde während 8 Stunden unter Argon im Metallbad auf 230°C erwärmt und ergab nach Filtration über eine Kieselgelsäule (Eluierungsmittel Hexan/AcOEt = 4:1, dann 1:1) und Umkristallisation aus EtOEt bei -78°C 7.3 g S-(3-Formylphenyl)dimethylthiocarbamat in goldgelben Kristallen, F.p. 76-77°C.

b) 13.2 g n-Heptyltriphenylphosphoniumbromid wurden in 200 ml THF suspendiert und bei -10°C tropfenweise mit 30 ml n-Butyllithium, 1.6 molar in Hexan, versetzt. Nach einstündigem Rühren bei 0°C erhielt man eine klare, rote Lösung, zu der eine Lösung von 6 g S-(3-Formylphenyl)dimethylthiocarbamat in 100 ml THF hinzugetropft wurde. Man rührte das Reaktionsgemisch 2 Stunden bei Raumtemperatur, goss auf ein Methanol/Wasser-Gemisch (6:4) und extrahierte mit Hexan. Die nicht-wässrige Phase wurde mehrfach mit Wasser gewaschen, getrocknet und eingedampft. Man erhielt nach Filtration über eine Kieselgelsäule (Eluierungsmittel Hexan/AcOEt = 9:1) 6.5 g S-(3-(1-Octenylphenyl)dimethylthiocarbamat als farbloses Oel (E/Z-Verhältnis ca. 1:2). 3 g dieses Produktes wurden in 250 ml Eisessig gelöst und nach Zugabe von 6 g Platinkohle (5%) bei 80°C/10 bar hydriert. Nach 1 Stunde wurde die Hydrierung abgebrochen, vom Katalysator abfiltriert und eingedampft. Nach Filtration über eine Kieselgelsäule (Eluierungsmittel Hexan/AcOEt = 9:1) erhielt man 2.4 g S-(3-Octylphenyl)dimethylthiocarbamat als farbloses Oel.

c) 0.4 g Lithiumaluminiumhydrid wurden in 25 ml THF suspendiert. Bei 0°C tropfte man eine Lösung von 2.4 g S-(3-Octylphenyl)dimethylthiocarbamat in 30 ml THF hinzu und rührte 2 Stunden bei 0°C. Anschliessend tropfte man eine Lösung von 1.2 g 3,3-Dimethylallylbromid in 10 ml THF hinzu und rührte weitere 2 Stunden bei 0°C. Das Reaktionsgemisch wurde auf Eiswasser gegossen, mit 6N Salzsäure angesäuert, mit EtOEt extrahiert, getrocknet und eingedampft. Nach Filtration über eine Kieselgelsäule (Eluierungsmittel Hexan/AcOEt = 4:1) erhielt man 2.3 g 3-Octylphenyl(3-methyl-2-butenyl)thioäther als farbloses Oel. Dieses Produkt wurde in 100 ml Toluol gelöst und nach Zugabe von 2 g p-Toluolsulfonsäure 20 Stunden am Wasserabscheider zum Rückfluss erhitzt. Nach dem Abkühlen neutralisierte man das Reaktionsgemisch durch Zugabe wässriger Natriumbicarbonat-Lösung und extrahierte mit AcOEt. Nach Filtration über eine Kieselgelsäule (Eluierungsmittel Hexan/AcOEt = 9:1) erhielt man 2.1 g 7-Octyl-3,4-dihydro-4,4-dimethyl-2H-1-benzothiopyran als schwach gelbliches Oel.

d) 0.6 g Acetylchlorid wurden in 50 ml Methylenchlorid gelöst und bei 0°C portionsweise mit 1 g Aluminiumchlorid versetzt. Nach 15 Minuten wurde eine Lösung von 2.1 g 7-Octyl-3,4-dihydro-4,4-dimethyl-2H-1-benzothiopyran in 50 mi Methylenchlorid hinzugetropft. Man liess 2 Stunden bei 0°C rühren, goss aus Eiswasser und extrahierte mit Methylenchlorid. Das Rohprodukt wurde durch Chromatographie an einer Lobar-Fertigsäule (Merck) gereinigt (Eluierungsmittel Hexan/AcOEt 1%). Man erhielt 1.3 g 6-Acetyl-7-octyl-3,4-dihydro-4,4-dimethyl-2H-1-benzothiopyran als leicht gelbliches Oel.

e) 1.3 g 6-Acetyl-7-octyl-3,4-dihydro-4,4-dimethyl-2H-1-benzothiopyran wurden in 100 ml Chloroform gelöst. Bei 0°C tropfte man zunächst 1 Aequivalent (0.74 g) 85% m-Chlorperbenzoesäure gelöst in 50 ml Chloroform dazu und nach 2 Stunden ein weiteres Aequivalent m-Chlorperbenzoesäure. Man rührte über Nacht bei 0°C, goss auf Eiswasser/Natriumcarbonat und extrahierte mit Methylenchlorid. Nach dem Umkristallisieren des Rohproduktes aus AcOEt/Hexan erhielt man 1 g 6-Acetyl-7-octyl-3,4-dihydro-4,4-dimethyl-2H-1-benzothiopyran-1,1-dioxid in farblosen Kristallen, F.p. 77-78°C.

f) In Analogie zu Beispiel 1 g) wurde durch Umsetzung von 0.95 g 6-Acetyl-7-octyl-3,4-dihydro-4,4-dimethyl-2H-1-benzothiopyran-1,1-dioxid mit 1.8 g Diäthyl (4-carbäthoxybenzyl)phosphonat nach Deprotonierung mit Natriumhydrid in Dimethylsulfoxid und Flashchromatographie des Rohproduktes an Kieselgel (Hexan/AcOEt = 1:1) 1.3 g Aethyl 4-[2-(4,4-dimethyl-7-octyl-1,1-dioxo-3,4-dihydro-2H-1-benzothiopyran-6-yl)-propenyl]-benzoat als schwach gelbes Oel isoliert mit einem E/Z-Verhältnis von ca. 4:9. Durch Bestrahlen des Rohproduktes mit einer Hg-Hochdrucklampe in THF während 5 Stunden lässt sich das E/Z-Verhältnis zugunsten des E-Isomeren verschieben (E/Z ~ 1:1). Mittels präparativer HPLC (Diisopropyläther/Hexan = 55:45) von 0.9 g E/Z-Gemisch erhielt man 300 mg des E-Isomeren und 430 mg des Z-Isomeren als schwach gelbliche Oele.

Beispiel 8

300 mg Aethyl (E)-4-[2-(4,4-dimethyl-7-octyl-1,1-dioxo-3,4-dihydro-2H-1-benzothiopyran-6-yl)-propenyl]-benzoat wurden in 30 ml Aethanol gelöst. Nach Zugabe einer Lösung von 330 mg Kaliumhydroxid in 10 ml Wasser erwärmte man 3 Stunden auf 40°C. Das klare Reaktionsgemisch wurde auf Eiswasser gegossen, mit 3N Salzsäure angesäuert und mehrfach mit AcOEt extrahiert. Nach Umkristalisation des Rohproduktes aus AcOEt/Hexan erhielt man 210 mg (E)-4-[2-(4,4-Dimethyl-7-octyl-1,1-dioxo-3,4-dihydro-2H-1-benzothiopyran-6-yl)-propenyl]-benzoesäure in weissen Kristallen, F.p. 176-178°C.

Beispiel 9

1.84 g 1-(1,1-Dioxo-5,5-dimethyl-8-octyloxy-2,3,4,5-tetrahydro-1-benzothiepin-7-yl)-äthanol wurden in 20 ml Acetonitril vorgelegt und mit 1.84 g Triphenylphosphinhydrobromid versetzt. Man erhitzte 65 Stunden unter Rückfluss, kühlte und dampfte im Vakuum ein. Der Rückstand wurde in $CH_2Cl_2$ aufgenommen, über $Na_2SO_4$ getrocknet und wieder eingedampft. Verrühren in 100 ml EtOEt/Hexan (1:1) lieferte schliesslich 2.82 g Phosphoniumsalz als weisse Kristalle, die wie folgt umgesetzt wurden:

Man löste sie unter Argon in 25 ml abs. THF und deprotonierte bei 0°C durch Zutropfen von 3.9 ml 1.55M nBuLi (Hexan). Zur roten Ylid-Lösung gab man nach 15 Minuten 814 mg 4-Formylbenzoesäureäthylester und liess 1 Stunde bei 0°C und 1 Stunde bei Raumtemperatur nachreagieren. Extraktion mit AcOEt, Waschen mit Wasser, Trocknen, Eindampfen, Flash-Chromatographie an Kieselgel (Hexan/AcOEt=85/15) und dreimaliges Umkristallisieren aus Hexan/AcOEt ergab schliesslich 702 mg (E)-4-[2-(8-Octyloxy-5,5-dimethyl-1,1-dioxo-2,3,4,5-tetrahydro-1-benzothiepin-7-yl)-propenyl]-benzoesäure-methylester als weisse Kristalle, F.p. 79-80°C.

Das als Ausgangsmaterial verwendete 1-(1,1-Dioxo-5,5-dimethyl-8-octyloxy-2,3,4,5-tetrahydro-1-benzothiepin-7-yl)-äthanol kann wie folgt hergerstellt werden:

a) 16.9 g Methoxymethyltriphenylphosphoniumchlorid wurden in 100 ml abs. THF vorgelegt und unter Argon bei -5°C langsam mit 30 ml 1.55M nBuLi versetzt. Nach 15 Minuten tropfte man eine Lösung von 8.26 g 8-Brom-2,3,4,5-tetrahydro-1-benzothiepin-5-on in 50 ml THF langsam zu und liess 2 Stunden nachreagieren. Dann verteilte man das Reaktionsgemisch zwischen Hexan und [EtOH/Wasser=8/2], trocknete die leichtere Phase und dampfte im Vakuum ein. Man erhielt 9.1 g rohes Enoläthergemisch, das in 70 ml THF unter Argon mit 70 ml 35% $HClO_4$ hydrolysiert wurde. Nach 2 Stunden bei Raumtemperatur wurde auf Eis gegossen, mit Wasser gewaschen, getrocknet und eingedampft. Flash-Chromatographie an Kieselgel (Hexan/AcOEt=95/5) ergab 6.86 g 8-Brom-2,3,4,5-tetrahydro-1-benzothiepin-5-carbaldehyd als farbloses Oel (91.5% rein nach GC).

b) 6.86 g des in a) erhaltenen Aldehyds wurden unter Argon in 70 ml abs. tBuOH vorgelegt und mit 5.76 g K-tert.-Butylat versetzt. Man kühlte auf ca. 15°C Innentemperatur und tropfte langsam 4.3 ml Methyljodid zu. Man rührte noch 2 Stunden, goss auf Eis, extrahierte mit EtOEt, wusch mit Wasser und NaCl-Lsg., trocknete und dampfte ein. Flash-Chromatographie an Kieselgel (Hexan/AcOEt=96/4) lieferte 3.24 g $\alpha$-methylierten Aldehyd als farbloses Oel.

c) Diese 3.24 g 8-Brom-5-methyl-2,3,4,5-tetrahydro-1-benzothiepin-5-carbaldehyd wurden in 45 ml Diäthylenglycol vorgelegt und mit 1.38 ml Hydrazinhydrat und 3.23 g KOH-Plätzchen versetzt. Man erwärmte zunächst 1 Stunde auf 100°C und anschliessend 3 Stunden auf 180°C. Nach Abkühlen goss man auf Eis, extrahierte mit EtOEt,

wusch mit Wasser, trocknete und dampfte im Vakuum ein. Flash-Chromatographie an Kieselgel (Hexan) ergab 2.58 g 8-Brom-5,5-dimethyl-2,3,4,5-tetrahydro-1-benzothiepin als farblose Kristalle, F.p. 81-82°C.

d) 2.54 g des obigen Bromids wurden unter Argon in 30 ml abs. THF vorgelegt und bei -78°C durch Zutropfen von 6.3 ml 1.5M nBuLi in die entsprechende Li-Verbindung überführt. Nach 30 Minuten gab man bei - 78°C 2.9 ml wasserfreies Nitrobenzol zu. Nach 1 Stunde goss man auf Eis, extrahierte mit EtOEt, wusch mit Wasser, trocknete und dampfte ein. Flash-Chromatographie an Kieselgel (Hexan/AcOEt, 95:5) lieferte 1.08 g 5,5-Dimethyl-2,3,4,5-tetrahydro-1-benzothiepin-8-ol als bräunliches Oel.

e) Unter Argon legte man in 10 ml abs. DMF 340 mg NaH (ca. 50%) vor. Bei 0°C tropfte man 1.08 g 5,5-Dimethyl-2,3,4,5-tetrahydro-1-benzothiepin-8-ol, gelöst in 10 ml abs. DMF, zu und rührte 30 Minuten. Dann gab man 1.36 g 1-Octylbromid zu und liess 2 Stunden bei Raumtemperatur reagieren. Man goss auf Eis, extrahierte mit EtOEt, wusch mit Wasser, trocknete und dampfte ein. Flash-Chromatographie an Kieselgel (Hexan/AcOEt=99/1) lieferte 1.60 g 5,5-Dimethyl-8-octyloxy-2,3,4,5-tetrahydro-1-benzothiepin als farbloses Oel.

f) Unter Argon legte man in 17 ml $CH_2Cl_2$ 0.86 ml AcCl und 1.24 g $AlCl_3$ vor. Bei -20°C tropfte man 1.60 g 5,5-Dimethyl-8-octyloxy-2,3,4,5-tetrahydro-1-benzothiepin, gelöst in 15 ml $CH_2Cl_2$ zu. Nach 15 Minuten goss man auf Eis, extrahierte mit EtOEt, wusch mit Bicarbonat- und NaCl-Lösung, trocknete und dampfte ein. Flash-Chromatographie an Kieselgel (Hexan/AcOEt=95/5) lieferte 1.97 g 1-(5,5-Dimethyl-8-octyloxy-2,3,4,5-tetrahydro-1-benzothiepin-7-yl)-äthanon als farbloses Oel.

g) 1.93 g 1-(5,5-Dimethyl-8-octyloxy-2,3,4,5-tetrahydro-1-benzothiepin-7-yl)-äthanon wurden in 40 ml $CH_2Cl_2$ vorgelegt und bei -25°C mit 3.81 g m-Chlorperbenzoesäure (ca. 85%-ig) versetzt. Man rührte 2.5 Stunden bei 0°C, goss auf Eis, extrahierte mit AcOEt, wusch nacheinander mit Pyrosulfit-Lsg., 2N NaOH, Wasser und NaCl-Lsg., trocknete und dampfte ein. Flash-Chromatographie an Kieselgel (Hexan/AcOEt=8/2) ergab 1.77 g 1-(1,1-Dioxo-5,5-dimethyl-8-octyloxy-2,3,4,5-tetrahydro-1-benzothiepin-7-yl)-äthanon als farbloses Oel.

h) 1.77 g 1-(1,1-Dioxo-5,5-dimethyl-8-octyloxy-2,3,4,5-tetrahydro-1-benzothiepin-7-yl)-äthanon wurden in 15 ml EtOH gelöst und mit 168 mg $NaBH_4$ versetzt. Nach 2 Stunden goss man auf Eis, extrahierte mit AcOEt, wusch mit Wasser, trocknete und dampfte ein. Dabei erhielt man 1.84 g DC-einheitliches 1-(1,1-Dioxo-5,5-dimethyl-8-octyloxy-2,3,4,5-tetrahydro-1-benzothiepin-7-yl)-äthanol als farbloses Oel.

Beispiel 10

In Analogie zu Beispiel 9 wurden hergestellt:

(E)-4-[2-(8-Hexyloxy-5,5-dimethyl-1,1-dioxo-2,3,4,5-tetrahydro-1-benzothiepin-7-yl)-propenyl]-benzoesäure-methylester als weisse Kristalle, F.p. 107.5-108-5°C;
(E)-4-[2-(8-Heptyloxy-5,5-dimethyl-1,1-dioxo-2,3,4,5-tetrahydro-1-benzothiepin-7-yl)-propenyl]-benzoesäure-methylester als weisse Kristalle, F.p. 92-93°C.

Beispiel 11

193 mg (E)-4-[2-(8-Heptyloxy-5,5-dimethyl-1,1-dioxo-2,3,4,5-tetrahydro-1-benzothiepin-7-yl)-propenyl]-benzoesäure-methylester wurden in 2 ml THF/EtOH (1/1) vorgelegt und mit 0.37 ml 3N NaOH versetzt. Man rührte über Nacht bei Raumtemperatur, goss auf Eis, extrahierte mit AcOEt, wusch mit wenig Wasser, trocknete und dampfte im Vakuum ein. Kristallisation aus AcOEt lieferte 152 mg (E)-4-[2-(8-Heptyloxy-5,5-dimethyl-1,1-dioxo-2,3,4,5-tetrahydro-1-benzothiepin-7-yl)-propenyl]-benzoesäure als weisse Kristalle, F.p. 153-154°C.

Beispiel 12

In Analogie zu Beispiel 11 wurden hergestellt:

(E)-4-[2-(8-Hexyloxy-5,5-dimethyl-1,1-dioxo-2,3,4,5-tetrahydro-1-benzothiepin-7-yl)-propenyl]-benzoesäure als weisse Kristalle, F.p. 157-158°C; und
(E)-4-[2-(8-Octyloxy-5,5-dimethyl-1,1-dioxo-2,3,4,5-tetrahydro-1-benzothiepin-7-yl)-propenyl]-benzoesäure als weisse Kristalle, F.p. 168-169°C.

Beispiel 13

3.58 g 1-(5,5-Dimethyl-8-octyl-1,1-dioxo-2,3,4,5-tetrahydro-1-benzothiepin-7-yl)-äthanol wurden in 20 ml Acetonitril vorgelegt und mit 3.34 g Triphenylphosphinhydrobromid versetzt. Man erhitzte 70 Stunden unter Rückfluss, kühlte und dampfte im Vakuum ein. Der Rückstand wurde in $CH_2Cl_2$ aufgenommen, über $Na_2SO_4$ getrocknet und wieder eingedampft. Verrühren in 200 ml EtOEt/Hexan (1:1) lieferte schliesslich 5.67 g Phosphoniumsalz als weisse Kristalle. Dieses Phosphoniumsalz wurde unter Argon in 80 ml abs. THF gelöst und bei 0°C durch Zutropfen von 7.45 ml 1.55M nBuLi deprotoniert. Nach 15 Minuten gab man 1.60 g 4-Formylbenzoesäure-methylester zu und liess 1 Stunde bei Raumtemperatur nachreagieren. Extraktion mit AcOEt, Waschen mit Wasser, Trocknen, Eindampfen, Flash-Chromatographie an Kieselgel (Hexan/AcOEt=80/20) und zweimaliges Umkristallisieren aus Hexan/AcOEt ergab schliesslich 1.41 g (E)-4-[2-(5,5-Dimethyl-8-octyl-1,1-dioxo-2,3,4,5-tetrahydro-1-benzothiepin-7-yl)-propenyl]-benzoesäure-methylester als weisse Kristalle, F.p. 85-86°C.

Das als Ausgangsmaterial verwendete 1-(5,5-Dimethyl-8-octyl-1,1-dioxo-2,3,4,5-tetrahydro-1-benzothiepin-7-yl)-äthanol wurde wie folgt hergestellt :

a) Aus 3.00 g 8-Brom-5,5-dimethyl-2,3,4,5-tetrahydro-1-benzothiepin und 340 mg Mg-Spänen bereitete man in 30 ml abs. THF unter Argon die entsprechende Grignard-Verbindung. Man kühlte auf -20°C und gab 220 mg gereinigtes CuI, gefolgt von 3.18 ml 1-Jodoctan, zu. Man erwärmte auf 0°C und goss nach 1.5 Stunden auf Eis/$NH_4Cl$-Lösung. Extraktion mit EtOEt, Waschen mit Wasser und NaCl-Lösung, Trocknen, Eindampfen und Flash-Chromatographie an Kieselgel (Hexan) lieferte 2.56 g 5,5-Dimethyl-8-octyl-2,3,4,5-tetrahydro-1-benzothiepin als farbloses Oel.

b) Dieses 5,5-Dimethyl-8-octyl-2,3,4,5-tetrahydro-1-benzothiepin wurde, wie unter Beispiel 9 f), g), und h) beschrieben, acetyliert, zum Sulfon oxydiert und schliesslich mit $NaBH_4$ zum 1-(5,5-Dimethyl-8-octyl-1,1-dioxo-2,3,4,5-tetrahydro-1-benzothiepin-7-yl)-äthanol reduziert.

Beispiel 14

In Analogie zu Beispiel 11 wurde hergestellt:

(E)-4-[2-(5,5-Dimethyl-8-octyl-1,1-dioxo-2,3,4,5-tetrahydro-1-benzothiepin-7-yl)-propenyl]-benzoesäure als weisse Kristalle, F.p. 164-165°C.

Beispiel 15

a) 2.20 g 1-(5,5-Dimethyl-8-hexyloxy-2,3,4,5-tetrahydro-1-benzothiepin-7-yl)-äthanon, hergestellt in Analogie zu Beispiel 9f), wurden in 20 ml abs. THF unter Argon vorgelegt und bei -20°C mit 9.9 ml 1M Vinylmagnesiumbromid-Lösung (THF) versetzt. Nach beendeter Zugabe (deutlich exotherm) liess man 1 Stunde nachreagieren und goss dann auf Eis/$NH_4Cl$. Extraktion mit EtOEt, Waschen mit gesättigter NaCl-Lösung, Trocknen und Eindampfen, gefolgt von Flash-Chromatographie an Kieselgel (Hexan(AcOEt = 95/5), ergab 2.16 g tertiären Alkohol als farbloses Oel.

b) Dieses wurde in 15 ml Acetonitril gelöst und mit 2.45 g Triphenylphosphinhydrobromid versetzt. Man rührte über Nacht bei Raumtemperatur, entfernte das LM im Vakuu und nahm den Rückstand in $CH_2Cl_2$ auf. Trocknen, Eindampfen und Digerieren in 200 ml EtOEt/Hexan = 1/1 lieferte 4.14 g umgelagertes Phosphoniumsalz als rosa Feststoff.

c) Diese 4.14 g wurden in 20 ml 1,2-Butylenoxyd vorgelegt und mit 691 mg (E)-3-Formyl-crotonsäureäthylester versetzt. Man erhitzte 1 Stunde zum Rückfluss, kühlte ab, goss auf Eis und extrahierte mit EtOEt. Waschen mit Wasser und gesättigter NaCl-Lösung, Trocknen, Eindampfen und Flash-Chromatographie an Kieselgel (Hexan/AcOEt = 96/4) lieferte 2.58 g Trienester, der folgendermassen mehrheitlich zur all-trans-Verbindung isomerisiert wurde:

d) Man löste diese 2.58 g unter Argon in 25 ml Acetonitril und gab 37 mg Pd(II)-nitrat, 134 mg Triphenylphosphin und 35 Mikroliter Triäthylamin zu. Man rührte 4 Stunden bei 50°C und arbeitete wieder auf. Erneute Flash-Chromatographie an Kieselgel (Hexan/AcOEt = 96/4) lieferte 2.33 g nahezu isomerenreinen (2E,4E,6E)-7-(8-Hexyloxy-5,5-dimethyl-2,3,4,5-tetrahydro-1-benzothiepin-7-yl)-3-methyl-octa-2,4,6-triensäureäthylester als blassgelbes Oel, das wie folgt zum Sulfon oxidiert wurde.

e) Man legte die Substanz in 75 ml $CH_2Cl_2$ vor und versetzte die Lösung bei -20°C mit 2.4 Eq. m-Chlorperbenzoesäure. Man liess auf 0°C erwärmen und verfolgte die Reaktion mittels Dünnschichtchromatographie. Nach 2 Stunden goss man auf Eis, extrahierte mit AcOEt, wusch nacheinander mit Na-Pyrosulfit-Lösung, 2N NaOH und NaCl-Lösung, trocknete und dampfte ein. Mitteldruckchromatographie an Kieselgel (Hexan/AcOEt = 85/15), gefolgt von Umkristallisation aus Hexan/AcOEt, lieferte schliesslich 760 mg (2E,4E,6E)-7-(8-Hexyloxy-5,5-dimethyl- 1,1-dioxo-2,3,4,5-tetrahydro-1-benzothiepin-7-yl)-3-methyl-octa-2,4,6-triensäureäthylester als farblose Kristalle vom Schmelzpunkt 116-120°C (dec.). Daneben fielen 170 mg des überoxydierten 6,7-Epoxyderivates an.

Beispiel 16

In Analogie zu Beispiel 15 wurde hergestellt:

(2E,4E,6E)-7-(8-Heptyloxy-5,5-dimethyl-1,1-dioxo-2,3,4,5-tetrahydro-1-benzothiepin-7-yl)-3-methyl-octa-2,4,6-triensäureäthylester als blass-gelbe Kristalle vom F.p. 98-99°C.

Beispiel 17

254 mg (2E,4E,6E)-7-(8-Hexyloxy-5,5-dimethyl- 1,1-dioxo-2,3,4,5-tetrahydro-1-benzothiepin-7-yl)-3-methyl-octa-2,4,6-triensäureäthylester wurden in 2 ml EtOH/THF = 1/1 vorgelegt und mit 0.5 ml 3N NaOH versetzt. Man rührte über Nacht, goss auf Eis, säuerte mit HCl conc. an, extrahierte mit AcOEt, wusch mit wenig Wasser, trocknete und dampfte im Vakuum ein. Kristallisation aus AcOEt/Hexan lieferte 139 mg (2E,4E,6E)-7-(8-Hexyloxy-5,5-dimethyl-1,1-dioxo-2,3,4,5-tetrahydro-1-benzothiepin-7-yl)-3-methyl-octa-2,4,6-triensäure als farblose Kristalle, F.p. 164-165°C.

Beispiel 18

In Analogie zu Beispiel 17 wurde hergestellt:

(2E,4E,6E)-7-(8-Heptyloxy-5,5-dimethyl-1,1-dioxo-2,3,4,5-tetrahydro-1-benzothiepin-7-yl)-3-methyl-octa-2,4,6-triensäure als blassgelbe Kristalle vom F.p. 152-153°C.

Beispiel 19

In Analogie zu Beispiel 15 wurde hergestellt:

(2E,4E,6E)-7-(5,5-Dimethyl-1,1-dioxo-2,3,4,5-tetrahydro-8-octyl-1-benzothiepin-7-yl)-3-methyl-octa-2,4,6-triensäureäthylester als farblose Kristalle vom F.p. 88-89°C.

Beispiel 20

In Analogie zu Beispiel 17 wurde hergestellt:

(2E,4E,6E)-7-(5,5-Dimethyl-1,1-dioxo-2,3,4,5-tetrahydro-8-octyl-1-benzothiepin-7-yl)-3-methyl-octa-2,4,6-triensäure als blass-gelbe Kristalle vom F.p. 104-105°C.

Beispiel 21

In Analogie zu Beispiel 9 wurde hergestellt:

(E)-4-[2-(6-Heptyloxy-3,3-dimethyl-1,1-dioxo-2,3-dihydro-benzo[b]-thiophen-5-yl)propenyl]-benzoesäuremethylester als weisse Kristalle vom F.p. 95-96°C.

Das als Ausgangsmaterial verwendete 6-Brom-3,3-dimethyl-2,3-dihydrobenzo[b]thiophen wurde wie folgt synthetisiert:

17,08 g 3-Bromthiophenol wurdenin 60 ml Aceton vorgelegt und bei 0°C mit 37,3 g gepulvertem $K_2CO_3$ versetzt. Dann tropfte man 10,1 ml Bromessigsäureäthylester langsam zu und liess 1 Stunde nachreagieren. Anschliessend goss man auf Eis, extrahierte mit EtOEt, wusch mit Wasser, trocknete über $Na_2SO_4$ und dampfte ein. Dabei erhielt man 24,05 g Produkt (GC >98%), das wie folgt weiterverarbeitet wurde:

Unter Argon bereitete man sich nach Standardverfahren in 150 ml abs. EtOEt aus 5,46 g Mg-Spänen und 14,5 ml MeI die entsprechende Grignard-Verbindung. Dazu tropfte man bei -10°C 22,8 g des oben hergestellten Esters, gelöst in 70 ml abs. EtOEt. Nach 1 Stunde goss man auf Eis/$NH_4Cl$, extrahierte mit EtOEt, wusch mit ges. NaCl-Lösung, trocknete über $Na_2SO_4$ und dampfte ein. Flash-Chromatographie an Kieselgel (Hexan/AcOEt = 85/15) ergab 11,94 g tertiären Alkohol, der folgendermassen cyclisiert wurde:

Unter Argon legte man in 80 ml $CS_2$ 21,7 g $AlCl_3$ vor. Unter Rühren tropfte man bei 0°C 11,94 g oben hergestellten tertiären Alkohol, gelöst in 10 ml $CS_2$, zu. Man erhitzte 3 Stunden zum Rückfluss, kühlte ab, goss vorsichtig auf Eis und extrahierte mit Hexan. Man wusch die organische Phase mit Wasser, trocknete über $Na_2SO_4$ und entfernte das Lösungsmittel i.V. Flash-Chromatographie an Kieselgel (Hexan) lieferte 9,54 g eines Gemisches, das nach GC 36,5% des gewünschten 6-Brom-3,3-dimethyl-2,3-dihydro-benzo[b]thiophens und 60% der regioisomeren 4-Brom-Verbindung enthielt. Die Trennung erfolgte auf der nächsten Stufe, nach Ueberführung in die entsprechenden Phenole, die wie unter 9d) beschrieben durchgeführt wurde.

Beispiel 22

In Analogie zu Beispiel 11 wurde hergestellt:

(E)-4-[2-(6-Heptyloxy-3,3-dimethyl-1,1-dioxo-2,3-dihydro-benzo[b]-thiophen-5-yl)propenyl]-benzoesäure als weisse Kristalle vom F.p. 151-152°C.

Beispiel A

Hartgelatinekapseln können wie folgt hergestellt werden:

| Bestandteile | mg/Kapsel |
|---|---|
| 1. sprühgetrocknetes Pulver enthaltend 75% Verbindung I | 20 |
| 2. Natriumdioctylsulfosuccinat | 0,2 |
| 3. Natriumcarboxymethylcellulose | 4,8 |
| 4. mikrokristalline Cellulose | 86,0 |
| 5. Talk | 8,0 |
| 6. Magnesiumstearat | 1.0 |
| Total | 120 |

Das sprühgetrocknete Pulver, das auf dem Wirkstoff, Gelatine und mikrokristalliner Cellulose basiert und eine mittlere Korngrösse des Wirkstoffes von <1 μ aufweist (mittels Autokorrelationsspektroskopie gemessen), wird mit einer wässrigen Lösung von Natriumcarboxymethylcellulose und Natriumdioctylsulfosuccinat befeuchtet und geknetet. Die resultierende Masse wird granuliert, getrocknet und gesiebt, und das erhaltene Granulat mit mikrokristallines Cellulose, Talk und Magnesiumstearat vermischt. Das Pulver wird in Kapseln der Grösse 0 abgefüllt.

Beispiel B

Tabletten können wie folgt hergestellt werden:

| Bestandteile | mg/Tablette |
|---|---|
| 1. Verbindung I als feingemahlenes Pulver | 20 |
| 2. Milchzucker pulv. | 100 |
| 3. Maisstärke weiss | 60 |
| 4. Povidone K30 | 8 |
| 5. Maisstärke weiss | 112 |
| 6. Talk | 16 |
| 7. Magnesiumstearat | 4 |
| Total | 320 |

Die feingemahlene Substanz wird mit Milchzucker und einem Teil der Maisstärke gemischt. Die Mischung wird mit einer wässrigen Lösung von Povidone K30 befeuchtet und geknetet, und die resultierende Masse granuliert, getrocknet und gesiebt. Das Granulat wird mit der restlichen Maisstärke, Talk und Magnesiumstearat vermischt und zu Tabletten geeigneter Grösse verpresst.

Beispiel C

Weichgelatinekapseln können wie folgt hergestellt werden:

| Bestandteile | mg/Kapsel |
|---|---|
| 1. Verbindung I | 5 |
| 2. Triglycerid | 450 |
| Total | 455 |

10 g Verbindung I werden unter Rühren, Inertbegasung und Lichtschutz in 90 g mittelkettigem Triglycerid gelöst. Diese Lösung wird als Kapselfüllmasse zu Weichgelatinekapseln à 5 mg Wirkstoff verarbeitet.

Beispiel D

Eine Lotion kann wie folgt hergestellt werden:

| Bestandteile | |
|---|---|
| 1. Verbindung I, feingemahlen | 1.0 g |
| 2. Carbopol 934 | 0,6 g |
| 3. Natriumhydroxid | q.s. ad pH 6 |
| 4. Aethanol, 94% | 50,0 g |
| 5. entmineralisiertes Wasser | ad 100,0 g |

Der Wirkstoff wird unter Lichtschutz in die Mischung Aethanol, 94%ig/Wasser eingearbeitet. Carbopol 934 wird bis zur vollständigen Gelierung eingerührt und der pH-Wert mit Natriumhydroxid eingestellt.

**Patentansprüche**

**1.** Verbindungen der Formel

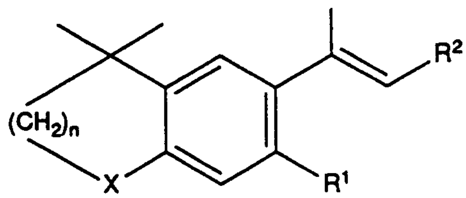

I

in der X -S-, -SO- oder $-SO_2-$;

$R^1$ $C_{7-10}$-Alkyl oder $C_{7-10}$-Alkoxy;
$R^2$ einen Rest der Formeln

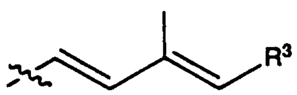

(a)

oder

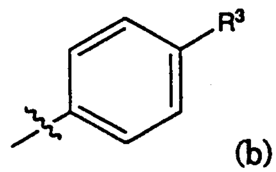

(b)

$R^3$ Carboxy oder $C_{1-6}$-Alkoxycarbonyl; und n 1,2 oder 3 darstellt; und Salze von Carbonsäuren der Formel I.

2. Verbindungen gemäss Anspruch 1, in denen $R^1$ Heptyl, Octyl, Heptyloxy oder Octyloxy ist.

3. Verbindungen gemäss den Ansprüchen 1 oder 2, in denen X = $SO_2$ ist.

4. Verbindungen gemäss den Ansprüchen 1-3, in denen $R^3$ Carboxyl ist.

5. Verbindungen gemäss den Ansprüchen 1-4, in denen $R^2$ ein Rest der Formel (a) ist.

6. Die Verbindungen gemäß Anspruch 5,

(all-E)-7-[7-Heptyloxy-3,4-dihydro-4,4-dimethyl-2H-1-benzothiopyran-6-yl]-3-methyl-octa-2,4,6-triensäureäthylester,
(all-E)-7-[7-Heptyloxy-3,4-dihydro-4,4-dimethyl-2H-1-benzothiopyran-6-yl]-3-methyl-2,4,6-octatriensäure,
(all-E)-7-[7-Heptyloxy-1-oxo-3,4-dihydro-4,4-dimethyl-2H-1-benzothiopyran-6-yl]-3-methyl-octa-2,4,6-triensäure,
(all-E)-7-[7'-(Heptyloxy)-3',4'-dihydro-4',4'-dimethyl-2'H-1-benzothiopyran-6'-yl)-3-methyl-2,4,6-octatriensäure-1',1'-dioxid,
(2E,4E,6E)-7-(8-Hexyloxy-5,5-dimethyl- 1,1-dioxo-2,3,4,5-tetrahydro-1-benzothiepin-7-yl)-3-methyl-octa-2,4,6-triensäureäthylester,
(2E,4E,6E)-7-(8-Heptyloxy-5,5-dimethyl-1,1-dioxo-2,3,4,5-tetrahydro-1-benzothiepin-7-yl)-3-methyl-octa-2,4,6-triensäureäthylester,
(2E,4E,6E)-7-(8-Hexyloxy-5,5-dimethyl-1,1-dioxo-2,3,4,5-tetrahydro-1-benzothiepin-7-yl)-3-methyl-octa-2,4,6-triensäure.

7. Die Verbindungen gemäß Anspruch 5,

(2E,4E,6E)-7-(8-Heptyloxy-5,5-dimethyl-1,1-dioxo-2,3,4,5-tetrahydro-1 benzothiepin-7-yl)-3-methyl-octa-2,4,6-triensäure,
(2E,4E,6E)-7-(5,5-Dimethyl-1,1-dioxo-2,3,4,5-tetrahydro-8-octyl-1-benzothiepin-7-yl)-3-methyl-octa-2,4,6-triensäureäthylester,
(2E,4E,6E)-7-(5,5-Dimethyl-1,1-dioxo-2,3,4,5-tetrahydro-8-octyl-1-benzothiepin-7-yl)-3-methyl-octa-2,4,6-triensäure.

8. Verbindungen gemäss den Ansprüchen 1-4, in denen $R^2$ ein Rest der Formel (b) ist.

9. Die Verbindungen gemäß Anspruch 8,

   Aethyl p-[(E,Z)-2-[3',4'-Dihydro-4',4'-dimethyl-7'-(heptyloxy)-2'H-1-benzothiopyran-6'-yl]propenyl]benzoat-1',1'-dioxid,
   p-[(E)-2-[3',4'-Dihydro-4',4'-dimethyl-7'-(heptyloxy)-2'H-1-benzothiopyran-6'-yl]propenyl]benzoesäure-1',1'-dioxid,
   p-[(Z)-2-[3',4'-Dihydro-4',4'-dimethyl-7'-(heptyloxy)-2'H-1-benzothiopyran-6'-yl]propenyl]benzoesäure-1',1'-dioxid,
   Aethyl-(E)4-[2-(4,4-dimethyl-7-octyl-1,1-dioxo-3,4-dihydro-2H-1-benzothiopyran-6-yl)-propenyl]-benzoat,
   Aethyl-(Z)4-[2-(4,4-dimethyl-7-octyl-1,1-dioxo-3,4-dihydro-2H-1-benzothiopyran-6-yl)-propenyl]-benzoat,
   (E)-4-[2-(4,4-Dimethyl-7-octyl-1,1-dioxo-3,4-dihydro-2H-1-benzothiopyran-6-yl)-propenyl]-benzoesäure,
   (E)-4-[2-(8-Octyloxy-5,5-dimethyl-1,1-dioxo-2,3,4,5-tetrahydro-1-benzothiepin-7-yl)-propenyl]-benzoesäure-methylester,
   (E)-4-[2-(8-Hexyloxy-5,5-dimethyl-1,1-dioxo-2,3,4,5-tetrahydro-1-benzothiepin-7-yl)-propenyl]-benzoesäure-methylester,
   (E)-4-[2-(8-Heptyloxy-5,5-dimethyl-1,1-dioxo-2,3,4,5-tetrahydro-1-benzothiepin-7-yl)-propenyl]-benzoesäure-methylester,
   (E)-4-[2-(8-Heptyloxy-5,5-dimethyl-1,1-dioxo-2,3,4,5-tetrahydro-1-benzothiepin-7-yl)-propenyl]-benzoesäure,
   (E)-4-[2-(8-Hexyloxy-5,5-dimethyl-1,1-dioxo-2,3,4,5-tetrahydro-1-benzothiepin-7-yl)-propenyl]-benzoesäure,
   (E)-4-[2-(8-Octyloxy-5,5-dimethyl-1,1-dioxo-2,3,4,5-tetrahydro-1-benzothiepin-7-yl)-propenyl]-benzoesäure,
   (E)-4-[2-(5,5-Dimethyl-8-octyl-1,1-dioxo-2,3,4,5-tetrahydro-1-benzothiepin-7-yl)-propenyl]-benzoesäure-methylester,
   (E)-4-[2-(5,5-Dimethyl-8-octyl-1,1-dioxo-2,3,4,5-tetrahydro-1-benzothiepin-7-yl)-propenyl]-benzoesäure.

10. Die Verbindungen gemäß Anspruch 8,

   (E)-4-[2-(6-Heptyloxy-3,3-dimethyl-1,1-dioxo-2,3-dihydro-benzo[b]-thiophen-5-yl)propenyl]-benzoesäuremethylester,
   (E)-4-[2-(6-Heptyloxy-3,3-dimethyl-1,1-dioxo-2,3-dihydro-benzo[b]-thiophen-5-yl)propenyl]-benzoesäure.

11. Verwendung von Verbindungen der Formel I von Anspruch 1 oder Salzen einer Carbonsäure der Formel I bei der Herstellung von pharmazeutischen Präparaten zur Behandlung von Autoimmunerkrankungen oder Erkrankungen mit einer stark immunologischen Komponente, insbesondere Psoriasis.

12. Pharmazeutische Präparate, enthaltend eine Verbindung der Formel I von Anspruch 1 oder Salzen einer Carbonsäure der Formel I und übliche pharmazeutische Trägerstoffe.

13. Verfahren zur Herstellung von Verbindungen der Formel I von Anspruch 1, dadurch gekennzeichnet, dass man

   a) eine Verbindung der Formel

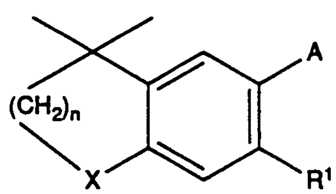

II

   mit einer Verbindung der Formeln

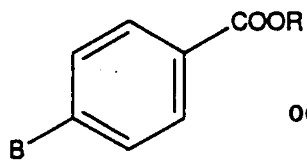

oder

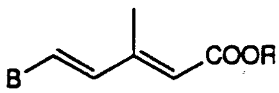

IV

III

umsetzt, wobei R $C_{1-6}$-Alkyl ist und entweder A eine Triphenylphosphoniumäthylgruppe

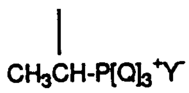

oder Dialkoxyphosphinyläthylgruppe

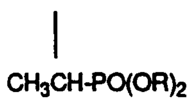

und B Formyl ist; oder A Acetyl und B eine Triphenylmethylphosphoniumgruppe $-CH_2$- $P[Q]_3^+ Y^-$ oder eine Dialkoxyphosphinylmethylgruppe $-CH_2-PO(OR)_2$ ist; wobei Q Phenyl ist; oder dass man
b) eine Verbindung der Formel

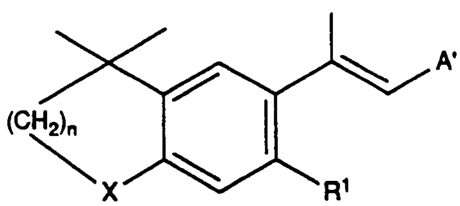

V

mit einer Verbindung der Formel

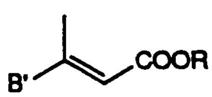

VI

wobei entweder A' eine Triphenylmethylphosphoniumgruppe $-CH_2$-$\overset{+}{P}[Q]_3Y^-$ oder eine Dialkoxyphosphinylmethylgruppe $-CH_2$-$PO(OR)_2$ und B' Formyl ist; oder A' Formyl und B' eine Triphenylmethylphosphoniumgruppe -$CH_2$-$\overset{+}{P}[Q]_3Y^-$ oder eine Dialkoxyphosphinylmethylgruppe $-CH_2PO(OR_2)$ ist, und wobei n, Q und R die oben angegebene Bedeutung haben,
zu einer Verbindung der Formel I, in der $R^3$ -COOR ist, umsetzt und gewünschtenfalls die Estergruppe -COOR verseift und die erhaltene Carbonsäure als solche oder als Salz isoliert; und/oder eine erhaltene Verbindung der Formel I, in der X oder -S- ist, zu einer Verbindung der Formel I, in der X -SO- oder $-SO_2$- ist, oxidiert.

## Claims

1. Compounds of the formula

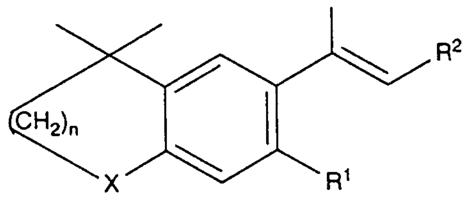

I

in which X represents -S-, -SO- or $-SO_2-$;

$R^1$ represents $C_{7-10}$-alkyl or $C_{7-10}$-alkoxy;
$R^2$ represents a residue of the formula

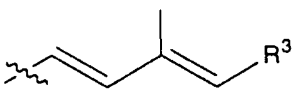

(a)

or

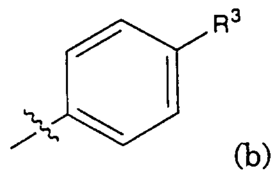

(b)

$R^3$ represents carboxy or $C_{1-6}$-alkoxycarbonyl; and n represents 1, 2 or 3; and salts of carboxylic acids of formula I.

2. Compounds according to claim 1, in which $R^1$ is heptyl, octyl, heptyloxy or octyloxy.

3. Compounds according to claim 1 or claim 2, in which X = $SO_2$.

4. Compounds according to any one of claims 1-3, in which $R^3$ is carboxyl.

5. Compounds according to any one of claims 1-4, in which $R^2$ is a residue of formula (a).

6. The compounds according to claim 5,

ethyl (all-E)-7-[7-heptyloxy-3,4-dihydro-4,4-dimethyl-2H-1-benzothiopyran-6-yl]-3-methyl-octa-2,4,6-trienoate,
(all-E)-7-[7-heptyloxy-3,4-dihydro-4,4-dimethyl-2H-1-benzothiopyran-6-yl]-3-methyl-2,4,6-octatrienoic acid,
(all-E)-7-[7-heptyloxy-1-oxo-3,4-dihydro-4,4-dimethyl-2H-1-benzothiopyran-6-yl]-3-methyl-octa-2,4,6-trienoic acid,
(all-E)-7-[7'-(heptyloxy)-3',4'-dihydro-4',4'-dimethyl-2'H-1-benzothiopyran-6'-yl)-3-methyl-2,4,6-octatrienoic acid 1',1'-dioxide,
ethyl (2E,4E,6E)-7-(8-hexyloxy-5,5-dimethyl- 1,1-dioxo-2,3,4,5-tetrahydro-1- benzothiepin-7-yl)-3-methyl-octa-2,4,6-trienoate,
ethyl (2E,4E,6E)-7-(8-heptyloxy-5,5-dimethyl-1,1-dioxo-2,3,4,5-tetrahydro-1-benzothiepin-7-yl)-3-methyl-octa-2,4,6-trienoate,
(2E,4E,6E)-7-(8-hexyloxy-5,5-dimethyl-1,1-dioxo-2,3,4,5-tetrahydro-1-benzothiepin-7-yl)-3-methyl-octa-2,4,6-trienoic acid.

7. The compounds according to claim 5,

(2E,4E,6E)-7-(8-heptyloxy-5,5-dimethyl-1,1-dioxo-2,3,4,5-tetrahydro-1-benzothiepin-7-yl)-3-methyl-octa-2,4,6-trienoic acid,
ethyl (2E,4E,6E)-7-(5,5-dimethyl-1,1-dioxo-2,3,4,5-tetrahydro-8-octyl-1-benzothiepin-7-yl)-3-methyl-octa-2,4,6-trienoate,
(2E,4E,6E)-7-(5,5-dimethyl-1,1-dioxo-2,3,4,5-tetrahydro-8-octyl-1-benzothiepin-7-yl)-3-methyl-octa-2,4,6-trienoic acid.

8. Compounds according to claims 1-4, in which $R^2$ is a residue of formula (b).

9. The compounds according to claim 8,

ethyl p-[(E,Z)-2-[3',4'-dihydro-4',4'-dimethyl-7'-(heptyloxy)-2'H-1-benzothiopyran-6'-yl]propenyl]benzoate 1',1'-dioxide,
p-[(E)-2-[3',4'-dihydro-4',4'-dimethyl-7'-(heptyloxy)-2'H-1-benzothiopyran-6'-yl]propenyl]benzoic acid 1',1'-dioxide,
p-[(Z)-2-[3',4'-dihydro-4',4'-dimethyl-7'-(heptyloxy)-2'H-1-benzothiopyran-6'-yl]propenyl]benzoic acid 1',1'-dioxide,
ethyl (E)4-[2-(4,4-dimethyl-7-octyl-1,1-dioxo-3,4-dihydro-2H-1-benzothiopyran-6-yl)-propenyl]-benzoate,
ethyl (Z)4-[2-(4,4-dimethyl-7-octyl-1,1-dioxo-3,4-dihydro-2H-1-benzothiopyran-6-yl)-propenyl]-benzoate,
(E)-4-[2-(4,4-dimethyl-7-octyl-1,1-dioxo-3,4-dihydro-2H-1-benzothiopyran-6-yl)-propenyl]-benzoic acid,
methyl (E)-4-[2-(8-octyloxy-5,5-dimethyl-1,1-dioxo-2,3,4,5-tetrahydro-1-benzothiepin-7-yl)-propenyl]-benzoate,
methyl (E)-4-[2-(8-hexyloxy-5,5-dimethyl-1,1-dioxo-2,3,4,5-tetrahydro-1-benzothiepin-7-yl)-propenyl]-benzoate,
methyl (E)-4-[2-(8-heptyloxy-5,5-dimethyl-1,1-dioxo-2,3,4,5-tetrahydro-1-benzothiepin-7-yl)-propenyl]-benzoate,
(E)-4-[2-(8-heptyloxy-5,5-dimethyl-1,1-dioxo-2,3,4,5-tetrahydro-1-benzothiepin-7-yl)-propenyl]-benzoic acid,
(E)-4-[2-(8-hexyloxy-5,5-dimethyl-1,1-dioxo-2,3,4,5-tetrahydro-1-benzothiepin-7-yl)-propenyl]-benzoic acid,
(E)-4-[2-(8-octyloxy-5,5-dimethyl-1,1-dioxo-2,3,4,5-tetrahydro-1-benzothiepin-7-yl)-propenyl]-benzoic acid,
methyl (E)-4-[2-(5,5-dimethyl-8-octyl-1,1-dioxo-2,3,4,5-tetrahydro-1-benzothiepin-7-yl)-propenyl]-benzoate,
(E)-4-[2-(5,5-dimethyl-8-octyl-1,1-dioxo-2,3,4,5-tetrahydro-1-benzothiepin-7-yl)-propenyl]-benzoic acid.

10. The compounds according to claim 8,

methyl (E)-4-[2-(6-heptyloxy-3,3-dimethyl-1,1-dioxo-2,3-dihydrobenzo[b]thiophen-5-yl)propenyl]-benzoate,
(E)-4-[2-(6-heptyloxy-3,3-dimethyl-1,1-dioxo-2,3-dihydrobenzo[b]-thiophen-5-yl)propenyl]benzoic acid.

11. The use of compounds of formula I of claim 1 or salts of a carboxylic acid of formula I for the manufacture of pharmaceutical preparations for the treatment of autoimmune diseases or disorders with a strong immunological component, especially psoriasis.

12. Pharmaceutical preparations containing a compound of formula I of claim 1 or salts of a carboxylic acid of formula I and usual pharmaceutical carrier materials.

13. A process for the manufacture of compounds of formula I of claim 1, characterized by

a) reacting a compound of the formula

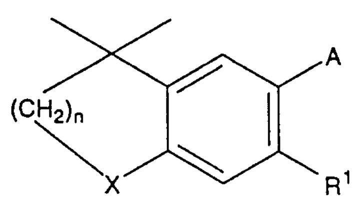

II

with a compound of the formula

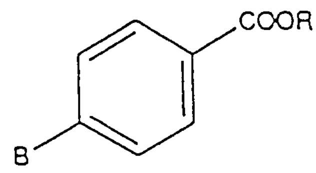

or

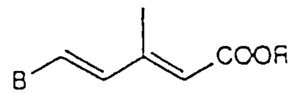

III IV

wherein R is $C_{1-6}$-alkyl and either A is a triphenylphosphoniumethyl group

$CH_3CH-P[Q]_3^+Y^-$

or dialkoxyphosphinylethyl group

$CH_3CH-PO(OR)_2$

and B is formyl; or A is acetyl and B is a triphenylmethylphosphonium group $-CH_2$. $P[Q]_3^+Y^-$ or a dialkoxyphosphinylmethyl group $-CH_2-PO(OR)_2$; and Q is phenyl; or

b) reacting a compound of the formula

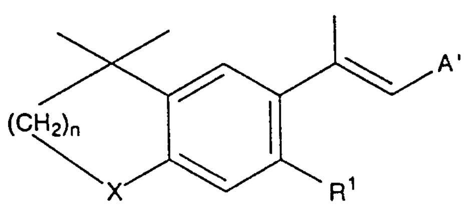

V

with a compound of the formula

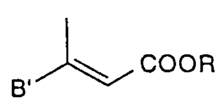

VI

wherein either A' is a triphenylmethylphosphonium group $-CH_2-\overset{+}{P}[Q]_3Y^-$ or a dialkoxyphosphinylmethyl group $-CH_2-PO(OR)_2$ and B' is formyl; or A' is formyl and B' is a triphenylmethylphosphonium group $CH_2-\overset{+}{P}[Q]_3Y^-$ or a dialkoxyphosphinylmethyl group $-CH_2PO(OR_2)$ and n, Q and R have the significance given above, to give a compound of formula I in which $R^3$ is -COOR and, if desired, saponifying the ester group -COOR and isolating the carboxylic acid obtained as such or as a salt; and/or oxidizing a compound of formula I obtained in which X is -S- to a compound of formula I in which X is -SO- or $-SO_2-$.

## Revendications

1. Composés de formule

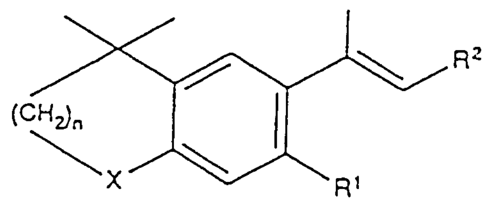

I

dans laquelle X est -S-, -SO- ou $-SO_2-$ ;

$R^1$ est un groupe alkyle en $C_{7-10}$ ou alcoxy en $C_{7-10}$ ;
$R^2$ est un radical de formules

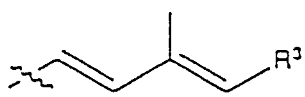

(a)

ou

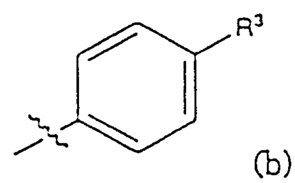

(b)

$R^3$ est un groupe carboxy ou (alcoxy en $C_{1-6}$)carbonyle ; et n vaut 1, 2 ou 3 ; et les sels d'acides carboxyliques de formule I.

2. Composés selon la revendication 1, dans lesquels $R^1$ est un groupe heptyle, octyle, heptyloxy ou octyloxy.

3. Composés selon les revendications 1 ou 2, dans lesquels X = $SO_2$.

4. Composés selon les revendications 1 à 3, dans lesquels $R^3$ est le groupe carboxyle.

5. Composés selon les revendications 1 à 4, dans lesquels $R^2$ est un radical de formule (a).

6. Composés selon la revendication 5, à savoir :

ester éthylique de l'acide (all-E)-7-[7-heptyloxy-3,4-dihydro-4,4-diméthyl-2H-1-benzothiopyranne-6-yl]-3-méthyl-octa-2,4,6-triénoïque,
acide (all-E)-7-[7-heptyloxy-3,4-dihydro-4,4-diméthyl-2H-1-benzothiopyranne-6-yl]-3-méthyl-2,4,6-octatriénoïque,
acide (all-E)-7-[7-heptyloxy-1-oxo-3,4-dihydro-4,4-diméthyl-2H-1-benzothiopyranne-6-yl]-3-méthyl-octa-2,4,6-triénoïque,
1',1'-dioxyde de l'acide (all-E)-7-[7'-heptyloxy-3',4'-dihydro-4',4'-diméthyl-2H-1-benzothiopyranne-6-yl]-3-méthyl-2,4,6-octatriénoïque,
ester éthylique de l'acide (2E,4E,6E)-7-(8-hexyloxy-5,5-diméthyl-1,1-dioxo-2,3,4,5-tétrahydro-1-benzothiépine-7-yl)-3-méthyl-octa-2,4,6-triénoïque,
ester éthylique de l'acide (2E,4E,6E)-7-(8-heptyloxy-5,5-diméthyl-1,1-dioxo-2,3,4,5-tétrahydro-1-benzothiépine-7-yl)-3-méthyl-octa-2,4,6-triénoïque,
acide (2E,4E,6E)-7-(8-hexyloxy-5,5-diméthyl-1,1-dioxo-2,3,4,5-tétrahydro-1-benzothiépine-7-yle)-3-méthylocta-2,4,6-triénoïque.

7. Composés selon la revendication 5, à savoir :

acide (2E,4E,6E)-7-(8-heptyloxy-5,5-diméthyl-1,1-dioxo-2,3,4,5-tétrahydro-1-benzothiépine-7-yl)-3-méthylocta-2,4,6-triénoïque,
ester éthylique de l'acide (2E,4E,6E)-7-(5,5-diméthyl-1,1-dioxo-2,3,4,5-tétrahydro-8-octyl-1-benzothiépine-7-yl)-3-méthyl-octa-2,4,6-triénoïque,
acide (2E,4E,6E)-7-(5,5-diméthyl-1,1-dioxo-2,3,4,5-tétrahydro-8-octyl-1-benzothiépine-7-yl)-3-méthyl-octa-2,4,6-triénoïque.

**8.** Composés selon les revendications 1 à 4, dans lesquels $R^2$ est un radical de formule (b).

**9.** Composés selon la revendication 8, à savoir

1',1'-dioxyde du p-[(E,Z)-2-[3',4'-dihydro-4',4'-diméthyl-7'-(heptyloxy)-2'H-1-benzothiopyranne-6'-yl]-propényl]benzoate d'éthyle,
1',1'-dioxyde de l'acide p-[(E)-2-[3',4'-dihydro-4',4'-diméthyl-7'-(heptyloxy)-2'H-1-benzothiopyranne-6'-yl]propényl]benzoïque,
1',1'-dioxyde de l'acide p-[(Z)-2-[3',4'-dihydro-4',4'-diméthyl-7'-(heptyloxy)-2'H-1-benzothiopyranne-6'- yl]propényl]benzoïque,
(E)-4-[2-(4,4-diméthyl-7-octyl-1,1-dioxo-3,4-dihydro-2H-1-benzothiopyranne-6-yl)-propényl]benzoate d'éthyle,
(Z)-4-[2-(4,4-diméthyl-7-octyl-1,1-dioxo-3,4-dihydro-2H-1-benzothiopyranne-6-yl)-propényl]benzoate d'éthyle,
acide (E)-4-[2-(4,4-diméthyl-7-octyl-1,1-dioxo-3,4-dihydro-2H-1-benzothiopyranne-6-yl)-propényl]benzoïque,
ester méthylique de l'acide (E)-4-[2-(8-octyloxy-5,5-diméthyl-1,1-dioxo-2,3,4,5-tétrahydro-1-benzothiépine-7-yl)-propényl]-benzoïque,
ester méthylique de l'acide (E)-4-[2-(8-hexyloxy-5,5-diméthyl-1,1-dioxo-2,3,4,5-tétrahydro-1-benzothiépine-7-yl)-propényl]-benzoïque,
ester méthylique de l'acide (E)-4-[2-(8-heptyloxy-5,5-diméthyl-1,1-dioxo-2,3,4,5-tétrahydro-1-benzothiépine-7-yl)-propényl]-benzoïque,
acide (E)-4-[2-(8-heptyloxy-5,5-diméthyl-1,1-dioxo-2,3,4,5-tétrahydro-1-benzothiépine-7-yl)-propényl]benzoïque,
acide (E)-4-[2-(8-hexyloxy-5,5-diméthyl-1,1-dioxo-2,3,4,5-tétrahydro-1-benzothiépine-7-yl)-propényl]benzoïque,
acide (E)-4-[2-(8-octyloxy-5,5-diméthyl-1,1-dioxo-2,3,4,5-tétrahydro-1-benzothiépine-7-yl)-propényl]benzoïque,
ester méthylique de l'acide (E)-4-[2-(5,5-diméthyl-8-octyl-1,1-dioxo-2,3,4,5-tétrahydro-1-benzothiépine-7-yl)-propényl]-benzoïque,
acide (E)-4-[2-(5,5-diméthyl-8-octyl-1,1-dioxo-2,3,4,5-tétrahydro-1-benzothiépine-7-yl)-propényl]benzoïque.

**10.** Composés selon la revendication 8, à savoir :

ester méthylique de l'acide (E)-4-[2-(6-heptyloxy-3,3-diméthyl-1,1-dioxo-2,3-dihydro-benzo[b]thiophène-5-yl)propényl]-benzoïque,
acide (E)-4-[2-(6-heptyloxy-3,3-diméthyl-1,1-dioxo-2,3-dihydro-benzo[b]thiophène-5-yl)propényl]-benzoïque.

**11.** Utilisation de composés de formule I selon la revendication 1 ou de sels d'un acide carboxylique de formule I pour fabriquer des préparations pharmaceutiques destinées au traitement de maladies auto-immunes ou de maladies ayant une forte composante immunologique, en particulier le psoriasis.

**12.** Préparations pharmaceutiques contenant un composé de formule I selon la revendication 1 ou des sels d'un acide carboxylique de formule I, et des supports pharmaceutiques usuels.

**13.** Procédé pour préparer des composés de formule I selon la revendication 1, caractérisé en ce que qu'on fait réagir

a) un composé de formule

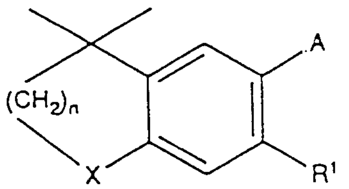

II

avec un composé de formules

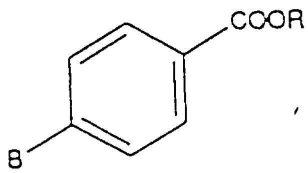

, ou

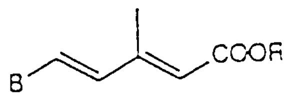

III

IV

où R est un groupe alkyle en $C_{1-6}$, et :

ou bien A est un groupe triphénylphosphoniuméthyle

$$CH_3CH-P[Q]_3^+Y^-$$

ou un groupe dialcoxyphosphinyléthyle

$$CH_3CH-PO(OR)_2$$

et B est le groupe formyle ;

ou bien A est le groupe acétyle et B est un groupe triphénylméthylphosphonium $-CH_2-P[Q]_3^+Y^-$ ou un groupe dialcoxyphosphinylméthyle $-CH_2-PO(OR)_2$ ; où Q est le groupe phényle ; ou encore en ce que

b) on fait réagir un composé de formule

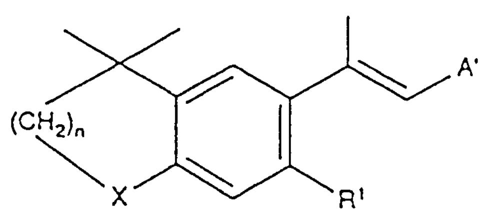

V

avec un composé de formule

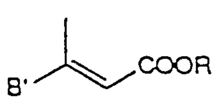

VI

dans laquelle :

ou bien A' est un groupe triphénylméthylphosphonium $-CH_2-P[Q]_3^+Y^-$ ou un groupe dialcoxyphosphinylméthyle $-CH_2-PO(OR)_2$ et B' est le groupe formyle ;

ou bien A' est le groupe formyle et B' est un groupe triphénylméthylphosphonium $-CH_2-P[Q]_3^+Y^-$ ou un groupe dialcoxyphosphinylméthyle $-CH_2PO(OR_2)$,

et où n, Q et R ont les significations données ci-dessus, pour donner un composé de formule I dans laquelle $R^3$ est -COOR, et, éventuellement, on saponifie le groupe ester -COOR et on isole l'acide carboxylique en tant que tel ou sous forme d'un sel ; et/ou on oxyde un composé ainsi obtenu de formule I dans laquelle X est -S-, pour donner un composé de formule I dans laquelle X est -SO-ou $-SO_2-$.